Europäisches Patentamt

(19) European Patent Office

Office européen des brevets

(11) Veröffentlichungsnummer: **0 048 396**
**B1**

(12) **EUROPÄISCHE PATENTSCHRIFT**

(45) Veröffentlichungstag der Patentschrift:
04.05.83

(21) Anmeldenummer: 81107114.1

(22) Anmeldetag: **10.09.81**

(51) Int. Cl.³: **C 07 C  51/27**, C 07 C  59/125

(54) Verfahren zur Herstellung von 2-Methoxyalkansäuren.

(30) Priorität: **20.09.80  DE 3035625**

(43) Veröffentlichungstag der Anmeldung:
**31.03.82 Patentblatt 82/13**

(45) Bekanntmachung des Hinweises auf die Patenterteilung:
**04.05.83 Patentblatt 83/18**

(84) Benannte Vertragsstaaten:
**CH DE FR LI SE**

(56) Entgegenhaltungen:
**DE-A-2 832 949**

(73) Patentinhaber: **BASF Aktiengesellschaft,
Carl-Bosch-Strasse 38, D-6700 Ludwigshafen (DE)**

(72) Erfinder: **Disteldorf, Walter, Dr., Portugieser Weg 17,
D-6706 Wachenheim (DE)**
Erfinder: **Eisfeld, Wolfgang, Dr., Dubliner Strasse 6,
D-6700 Ludwigshafen (DE)**

## Verfahren zur Herstellung von 2-Methoxyalkansäuren

Die Erfindung betrifft ein Verfahren zur Herstellung von 2-Methoxyalkansäuren mit 2 bis 5 C-Atomen durch Oxidation der entsprechenden 2-Methoxyalkanole mit Salpetersäure.

In der DE-OS 28 32 949 wird ein Verfahren zur Herstellung von Methoxyessigsäure beschrieben, bei dem man 2-Methoxyethanol in Gegenwart eines Vanadiumkatalysators mit Salpetersäure oxidiert. Bei diesem bekannten Verfahren, bei dem man 3 bis 5 Mol Salpetersäure je Mol 2-Methoxyethanol anwendet, erwärmt man die Salpetersäure mit einem Teil des 2-Methoxyethanols zur Induzierung der Reaktion kurzfristig auf 90° C, wonach die Oxidation bei 40 bis 60° C zu Ende geführt wird. Vor Isolierung der Methoxyessigsäure wird der größte Teil der nicht umgesetzten Salpetersäure im Vakuum abdestilliert. Dann gibt man Formaldehyd oder Paraformaldehyd hinzu und reduziert anschließend die noch vorhandene salpetrige Säure bzw. Stickoxide durch Zugabe von Harnstoff.

Dieses Verfahren bietet bei der technischen Durchführung Risiken. So geht bei Beginn der Reaktion die Oxidation bei einer Temperatur von etwa 90° C über die Stufe der Methoxyessigsäure hinaus. Andestillieren nach der Oxidation unter Vakuum führt zunächst zu einer vollständigen Entwässerung des Reaktionsgemisches und damit zu einer Anreicherung von hochkonzentrierter Salpetersäure im Sumpf, wodurch detonationsfähige Gemische entstehen. Versucht man, die salpetersauren Oxidationsgemische drucklos zu destillieren, so kann man zwar bis zu einer Sumpftemperatur von 150° C die Salpetersäure gefahrlos entfernen, hat hierbei jedoch hohe Ausbeuteverluste durch Überoxidation hinzunehmen. Außerdem weist die so erhaltene Rohsäure einen N-Gehalt von etwa 0,3% auf.

Es wurde nun gefunden, daß man 2-Methoxyalkansäuren mit 2 bis 5 C-Atomen durch Oxidation der entsprechenden 2-Methoxyalkanole mit Salpetersäure in Gegenwart von Vanadationen als Katalysator bei Temperaturen unter 90° C und destillative Abtrennung der 2-Methoxyalkansäure mit hoher Selektivität und technischer Sicherheit herstellen kann, wenn man

(a) weniger als 3 Mol Salpetersäure, bezogen auf 1 Mol 2-Methoxyalkanol, verwendet,
(b) die Oxidation bei Temperaturen von 40 bis 80° C durchführt,
(c) das Reaktionsgemisch nach Zugabe eines Überschusses an Formaldehyd, Paraformaldehyd, Trioxan oder Ameisensäure zunächst auf 60 bis 100° C und dann unter Abdestillieren von Wasser ohne Vakuum auf Temperaturen von über 100° C erhitzt und
(d) den Rückstand nach Isolierung der 2-Methoxyalkansäure durch fraktionierte Destillation erneut als Katalysator für die Oxidation von 2-Methoxyalkanol nach diesem Verfahren einsetzt.

Nach dem neuen Verfahren werden 2-Methoxyalkanole mit 2 bis 5 C-Atomen, wie 2-Methoxyethanol oder 2-Methoxypropanol, mit weniger als 3 Mol, vorzugsweise 2,5 bis 1,8 Mol Salpetersäure, bezogen auf ein Mol Methoxyalkanol oxidiert.

Als Salpetersäure verwendet man zweckmäßigerweise 30- bis 80%ige wäßrige Salpetersäure, die bei der diskontinuierlichen Arbeitsweise vorzugsweise insgesamt vorgelegt wird.

Die Oxidation führt man bei Temperaturen von 40 bis 80° C durch. Zum verzögerungsfreien Starten der Oxidation hat es sich als vorteilhaft erwiesen, das zum Einleiten der Reaktion benötigte freie $NO_2$ durch Zugabe von Formaldehyd, Paraformaldehyd oder Trioxan bei Temperaturen von 40 bis 60° C zur vorgelegten Salpetersäure zu erzeugen. Da hierbei die Menge an freiem $NO_2$ 0,1 bis 2, vorzugsweise 0,3 bis 0,9 Gewichtsprozent, bezogen auf die wäßrige Salpetersäure, betragen sollte, gibt man, bezogen auf die wäßrige Salpetersäure, 0,03 bis 1, vorzugsweise 0,09 bis 0,4 Gewichtsprozent an Formaldehyd, Paraformaldehyd oder Trioxan zur vorgelegten Salpetersäure.

Als Katalysatoren verwendet man Vanadationen enthaltende Katalysatoren. Man gibt sie, z. B. in Form von Ammoniumvanadat, Vanadinpentoxid oder Vanadinoxidsulfat zur vorgelegten Salpetersäure. Besonders vorteilhafte Ergebnisse werden erzielt, wenn man Katalysatoren verwendet, die Vanadat- und Kupferionen enthalten. Kupferionen werden z. B. in Form von Kupfersalzen, wie Kupferacetat, -carbonat oder -nitrat oder als metallisches Kupfer zugegeben. Der Katalysator wird z. B. in einer Menge von 0,003 bis 0,3, vorzugsweise 0,03 bis 0,07 Gewichtsprozent (berechnet auf V und Cu), bezogen auf die wäßrige Salpetersäure, eingesetzt. Das g-Atomverhältnis Vanadium zu Kupfer kann z. B. 5 : 1 bis 1 : 8 betragen. Besonders günstig hat sich ein g-Atomverhältnis von 1 : 1 bis 1 : 4 erwiesen.

Die Oxidation selbst wird in einem Temperaturbereich von 40 bis 80° C drucklos durchgeführt, wobei man bis zum 85%igen Umsatz eine Temperatur von 60° C nicht überschreiten sollte. Zur Nachoxidation kann dann die Temperatur auf 80° C erhöht werden.

Nach der Erfindung wird das Reaktionsgemisch dadurch aufgearbeitet, daß man zum Abbau restlicher Salpetersäure einen Überschuß an Formaldehyd, Paraformaldehyd, Trioxan oder Ameisensäure hinzugibt. Die erforderlichen Mengen sind pro Mol noch vorhandener Salpetersäure 0,5 bis 1,5, vorzugsweise 0,7 bis 0,9 Mol Formaldehyd, oder die entsprechende Menge der Polymeren, oder 0,8 bis 2,4, vorzugsweise 1,2 bis 1,6 Mol Ameisensäure.

Formaldehyd wird zweckmäßig als etwa 40%ige wäßrige Lösung eingesetzt. Diese Behandlung wird bei Temperaturen von 60 bis 100°C, vorzugsweise 85 bis 95°C durchgeführt. Die Behandlungsdauer beträgt mindestens 30 Minuten, vorzugsweise etwa 1 bis 3 Stunden. Nach dieser Behandlung liegt der Salpetersäuregehalt des Reaktionsgemisches bei Werten unter 1 Gewichtsprozent. Nun wird unter gleichzeitigem Abdestillieren von Wasser ohne Vakuum auf Temperaturen über 100°C, beispielsweise auf 100 bis 110°C, erhitzt, wobei man als Rückstand ein Konzentrat mit hohem Gehalt an 2-Methoxyalkansäure erhält, das überraschenderweise einen Salpetersäuregehalt von unter 0,01 Gewichtsprozent aufweist. Bei Verwendung eines Vanadationen oder Kupfer- und Vanadationen enthaltenden Katalysators ist diese weitgehende Zerstörung der Salpetersäure an einem Farbumschlag von blaugrün nach tiefblau zu erkennen, der durch Reduktion des Vanadats zum $VO^{2+}$-Ion bedingt ist.

Zur Isolierung der 2-Methoxyalkansäure wird der flüssige Rückstand aus der vorangegangenen Aufkonzentrierung fraktioniert destilliert, vorzugsweise im Vakuum. Im Destillationssumpf verbleibt die gesamte eingesetzte Vanadium- und Kupfermenge. Ohne weitere Behandlung wird der Sumpf in Salpetersäure aufgenommen und ohne Zugabe eines Katalysators für eine erneute Oxidation eingesetzt. Selbst nach 10maliger Recyclisierung ist weder ein Ausbeuteverlust an 2-Methoxyalkansäure noch eine Gewichtszunahme beim Destillationssumpf festzustellen. Der N-Gehalt des Sumpfes liegt stets < 0,1% und bildet damit kein Sicherheitsrisiko.

Man kann das neue Verfahren sowohl diskontinuierlich als auch kontinuierlich durchführen. Hierbei richtet sich die Dosierrate des 2-Methoxyalkanols nach den Möglichkeiten der Wärmeabfuhr. Durch den Einsatz von weniger als 3 Mol Salpetersäure pro Mol 2-Methoxyalkanol erhält man nach der 2-Methoxyalkanol-Zugabe zunächst eine Oxidationslösung, die bis zu 15% des angesetzten Methylglykols in Form von 2-Methoxyalkyl-2-methoxyalkanat enthält. Dieser Ester wird jedoch unter den Reaktionsbedingungen bei Temperaturen von 60 bis 80°C verseift, wobei das freigesetzte 2-Methoxyalkanol zur gewünschten Säure aufoxidiert wird. Zu diesem Zeitpunkt ist die Salpetersäure-Konzentration in der Oxidationslösung soweit vermindert, daß eine merkliche saure Etherspaltung, selbst bei Temperaturanhebung bis auf 80°C, unterbleibt.

Nach dem erfindungsgemäßen Verfahren werden die 2-Methoxyalkansäuren in hoher Ausbeute völlig gefahrlos und technisch besonders einfach erhalten. Darüber hinaus bietet die beliebige Wiederverwendung des Sumpfes aus der fraktionierten Destillation als Katalysator für neue Ansätze einen unerwarteten Vorteil, besonders auch im Hinblick auf die damit erheblich verminderte Umweltbelastung.

Die 2-Methoxyalkansäuren, insbesondere die Methoxyessigsäure, sind z. B. als Hilfsmittel in der Textilindustrie oder als Zwischenprodukte für die Herstellung von Fungiziden anwendbar.

### Beispiel 1

In einem Rührbehälter aus salpetersäurebeständigen Material, der mit Heiz- und Kühlvorrichtung, Rückflußkühler und Abgasleitung zur Nitrosevernichtung versehen ist, werden 3500 Teile 63%ige Salpetersäure, 1,75 Teile $NH_4VO_3$ und 3,85 Teile $Cu(Ac)_2 \cdot H_2O$ vorgelegt. Das g-Atomverhältnis Vanadium zu Kupfer beträgt in diesem Fall 1 zu 1,6. Man erwärmt auf 55°C und gibt unter Rühren 14 Teile einer 40%igen wäßrigen Formaldehyd-Lösung (das sind 0,16 Gewichtsprozent Formaldehyd, bezogen auf die 63%ige Salpetersäure) hinzu, wobei sich die Lösung durch $NO_2$-Bildung dunkelbraun färbt. Über einen Zeitraum von 4 bis 6 Stunden werden 1064 Teile 2-Methoxyethanol so zugegeben, daß eine Temperatur von 50 bis 55°C eingehalten wird. Das Molverhältnis Salpetersäure zu 2-Methoxyethanol beträgt in diesem Beispiel 2,5 zu 1. Das Reaktionsgemisch wird nach der Zugabe des Methoxyethanols noch 1 Stunde bei 60°C gehalten. Wie sich durch GC-Analyse feststellen läßt, sind 85% des 2-Methoxyethanols oxidiert worden. Man oxidiert nach, indem man das Gemisch 3 Stunden auf 80°C hält. Zur Zerstörung der Salpetersäure werden nun bei 90 bis 95°C innerhalb von 1 bis 2 Stunden 390 Teile einer wäßrigen 40%igen Formaldehyd-Lösung (das sind 0,7 Mol Formaldehyd, bezogen auf 1 Mol Salpetersäure im Oxidationsaustrag) gleichmäßig zugegeben. Danach wird noch 1 Stunde unter Rückfluß gerührt. Dann wird zunächst bei Normaldruck bis zu einer Sumpftemperatur von 110°C Wasser abdestilliert, wobei man als Sumpf eine dunkelblaue Lösung erhält, die < 0,01 Gewichtsprozent Salpetersäure, < 0,1 Gewichtsprozent Glykolsäure und < 0,1 Gewichtsprozent Oxalsäure enthält. Schließlich wird bei 100 mbar fraktionierend destilliert. Es werden 1115 Teile Methoxyessigsäure mit einem Wassergehalt von unter 0,2 Gewichtsprozent erhalten. Die Ausbeute beträgt 88,1 Molprozent. Der den Katalysator enthaltende Destillationssumpf (14—17 Teile) wird ohne Aufarbeitung für einen erneuten Einsatz gemäß diesem Beispiel verwendet, wobei auch nach 10maliger Recyclisierung gütegleiche Methoxyessigsäure in gleicher Ausbeute und eine etwa gleichbleibende Destillationssumpfmenge gewonnen werden.

### Beispiel 2

Man verfährt wie im Beispiel 1 beschrieben, wobei man jedoch anstelle von 1064 Teilen 2-Methoxyethanol 1260 Teile 2-Methoxypropanol einsetzt. Man erhält 1134 Teile 2-Methoxypropionsäure mit einem Wassergehalt von unter 0,2 Gewichtsprozent; Ausbeute 77,8 Molprozent.

Der den Katalysator enthaltene Destillationssumpf ist gemäß Beispiel 1 rückführbar.

## Patentansprüche

1. Verfahren zur Herstellung von 2-Methoxyalkansäuren mit 2 bis 5 C-Atomen durch Oxidation der entsprechenden 2-Methoxyalkanole mit Salpetersäure in Gegenwart von Vanadationen als Katalysator bei Temperaturen unter 90°C und destillative Abtrennung der 2-Methoxyalkansäure, dadurch gekennzeichnet, daß man

(a) weniger als 3 Mol Salpetersäure, bezogen auf 1 Mol Methoxyalkanol, verwendet,
(b) die Oxidation bei Temperaturen von 40 bis 80°C durchführt,
(c) das Reaktionsgemisch nach Zugabe eines Überschusses an Formaldehyd, Paraformaldehyd, Trioxan oder Ameisensäure zunächst auf 60 bis 100°C und dann unter Abdestillieren von Wasser ohne Vakuum auf Temperaturen von über 100°C erhitzt und
(d) den Rückstand nach Isolierung der 2-Meth-oxyalkansäure durch fraktionierte Destilla-tion erneut als Katalysator für die Oxidation von 2-Methoxyalkanol nach diesem Verfahren einsetzt.

2. Verfahren nach Anspruch 1, dadurch gekennzeichnet, daß man 2,5 bis 1,8 Mol Salpetersäure pro Mol 2-Methoxyalkanol anwendet.

3. Verfahren nach Anspruch 1, dadurch gekennzeichnet, daß man die Salpetersäure und den Katalysator vorlegt und vor der Zugabe des 2-Methoxyalkanols 0,03 bis 1 Gewichtsprozent, bezogen auf die wäßrige Salpetersäure, an Formaldehyd, Paraformaldehyd oder Trioxan bei Temperaturen von 40 bis 60°C zur vorgelegten Salpetersäure gibt.

4. Verfahren nach Anspruch 1, dadurch gekennzeichnet, daß man einen Katalysator verwendet, der Vanadat- und Kupferionen enthält.

## Claims

1. A process for the preparation of a 2-methoxyalkanoic acid of 2 to 5 carbon atoms by oxidizing the corresponding 2-methoxyalkanol with nitric acid in the presence of vanadate ions as the catalyst, at below 90°C, and isolating the 2-methoxyalkanoic acid by distillation, wherein

(a) less than 3 moles of nitric acid are used per mole of methoxyalkanol,
(b) the oxidation is carried out at from 40 to 80°C,
(c) the reaction mixture is heated, after the addition of an excess of formaldehyde, paraformaldehyde, trioxane or formic acid, first at 60—100°C and then, whilst distilling off water but without applying reduced pressure, at above 100°C, and
(d) the residue left after isolating the 2-methoxyalkanoic acid by fractional distillation, is re-used as a catalyst for oxidizing the 2-methoxyalkanol by the above process.

2. A process as claimed in claim 1, wherein from 2.5 to 1.8 moles of nitric acid are employed per mole of 2-methoxyalkanol.

3. A process as claimed in claim 1, wherein the nitric acid and the catalyst are initially charged into the reactor and, before addition of the 2-methoxyalkanol, from 0.03 to 1 per cent by weight, based on the aqueous nitric acid, of formaldehyde, paraformaldehyde or trioxane are added, at from 40 to 60°C, to the nitric acid in the reactor.

4. A process as claimed in claim 1, wherein a catalyst which contains vanadate ions and copper ions is employed.

## Revendications

1. Procédé de préparation d'acides 2-méth-oxyalcanoïques en C2—C5 par oxydation des 2-méthoxyalcanols correspondants par l'acide nitrique en présence d'ions vanadate servant de catalyseur à des températures inférieures à 90°C avec séparation de l'acide 2-méthoxyalcanoïque par distillation, caractérisé en ce que:

a) on utilise moins de 3 moles d'acide nitrique pour 1 mole de méthoxyalcanol,
b) on effectue l'oxydation à des températures de 40 à 80°C,
c) on chauffe le mélange de réaction, après addition d'un excès de formaldéhyde, de paraformaldéhyde, de trioxanne ou d'acide formique, d'abord à une température de 60 à 100°C puis, en distillant l'eau sans application du vide, à des températures supérieures à 100°C, et
d) après isolement de l'acide 2-méthoxyalcanoïque par distillation fractionnée, on réutilise le résidu en tant que catalyseur d'oxydation d'un 2-méthoxyalcanol par le même procédé.

2. Procédé selon la revendication 1, caractérisé en ce que l'on utilise 2,5 à 1,8 moles d'acide nitrique par mole de 2-méthoxyalcanol.

3. Procédé selon la revendication 1, caractérisé en ce que l'on introduit d'abord dans l'appareil l'acide nitrique et le catalyseur et, avant d'ajouter le 2-méthoxyalcanol, on ajoute à l'acide nitrique 0,03 à 1% en poids, par rapport à l'acide nitrique aqueux, de formaldéhyde, de paraformaldéhyde ou de dioxanne à des températures de 40 à 60°C.

4. Procédé selon la revendication 1, caractérisé en ce que l'on utilise un catalyseur qui contient des ions vanadate et cuivre.